# EUROPEAN PATENT APPLICATION

(11) **EP 4 375 361 A1**
(43) Date of publication of application: **29.05.2024**
(21) Application number: 23208568.8
(22) Date of filing: 08.11.2023
(51) Int. Cl.: C12M 1/16, C12M 1/34

(54) **AIR FLOW TYPE SOLID STATE CULTURE APPARATUS AND AIR FLOW TYPE SOLID STATE CULTURE METHOD**

(30) Priority: 24.11.2022 JP 2022187060
(71) Applicant: Fujiwara Techno-Art Co., Ltd., Okayama 701-1133 (JP)
(72) Inventor: Kariyama, Masahiro, Okayama-shi, Okayama (JP); Okamoto, Toshihiro, Okayama-shi, Okayama (JP); Harada, Toshiyasu, Okayama-shi, Okayama (JP); Wakimoto, Katsumasa, Okayama-shi, Okayama (JP); Tokuyama, Mako, Okayama-shi, Okayama (JP)
(74) Representative: Winter, Brandl - Partnerschaft mbB

(57) **Abstract**

An air flow type solid state culture apparatus (1) cultures microorganisms while adjusting temperature and humidity of ambient air taken into the apparatus, the air flow type solid state culture apparatus (1) includes an air intake (2), a blower (3), an air conditioning mechanism (4), a culture room (5), an air outlet (6), and a control device (8), the air conditioning mechanism (4) is constituted by a cooling unit (41), a heating unit (42), a water spray unit (43), and a steam supply unit (44), when the set values for the temperature and humidity of the adjusted air are specified, among the units (41) to (44) constituting the air conditioning mechanism (4), the control device (8) selects a combination of the units suitable for causing the temperature and humidity of preadjustment air to reach the set values.

## Description

### TECHNICAL FIELD

The present invention relates to an air flow type solid state culture apparatus and an air flow type solid state culture method that culture microorganisms while taking ambient air into the apparatus, and exhausting all the air that has passed through a culture substrate to the outside of the apparatus.

### BACKGROUND

A solid state culture apparatus is generally operated by circulating blowing air within the apparatus. However, for example, in production of a seed koji or enzyme, when there are many miscellaneous bacteria, the degradation of a product will be caused. Therefore, an air conditioning control system called a one-way system, in which all the air that has passed through a culture substrate is exhausted to the outside of the apparatus without circulating blowing air within the apparatus, was optimal.

In a culture apparatus illustrated in Fig. 2 of JP 9-154569 A, there is no inner air circulation path, and air (ambient air) to be sent into a culture room is blown from a blower into a lower room via a blowing-in duct through an air conditioning apparatus, passes through a culture bed, and is exhausted from an exhaust duct provided in an upper room (paragraph [0018]).

Additionally, in a conventional solid state culture apparatus, when adjusting the temperature and humidity of the air to be supplied to a culture substrate, units are fixed according to a purpose of adjustment, such as a heating unit is used for heating, a cooling unit is used for cooling, a water spray unit is used for humidification, and the cooling unit and the heating unit are used for dehumidification. Further, although there has been a solid state culture apparatus that includes a steam supply unit for a purpose of sterilizing of the apparatus, the steam supply unit has not been used as a unit for adjusting the air to be supplied to the culture substrate while performing solid state culture.

In a culture apparatus illustrated in Fig. 1 of JP 4316021 B, air (ambient air) taken in from an air intake is ventilated from a bottom of a container to inside of the culture apparatus through a cooler of an air conditioner, etc., and the ventilation is released to the outside from an outlet of a canopy through a flowmeter, without circulation (paragraph [0019]).

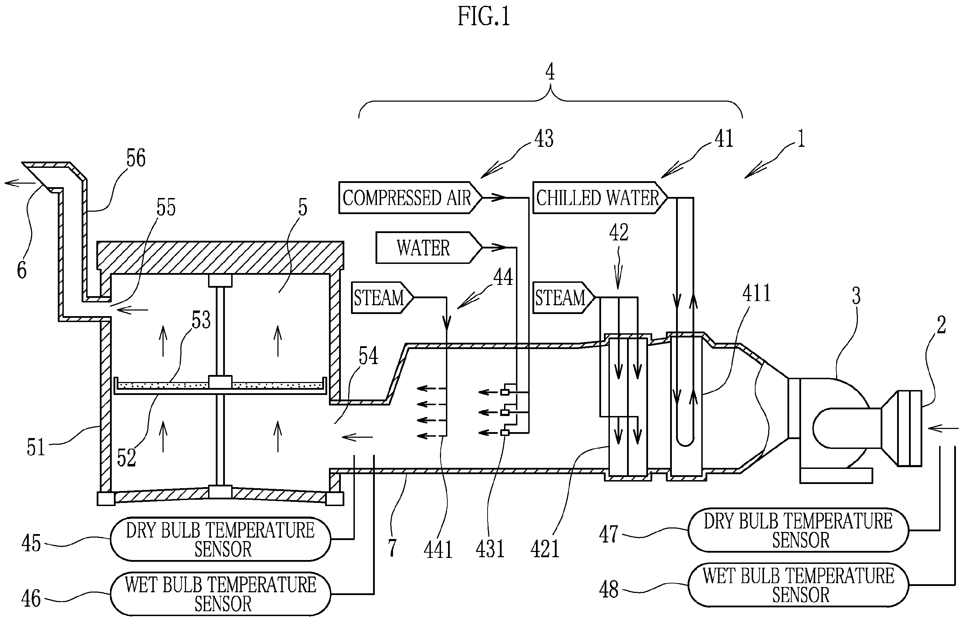

Although a koji making apparatus illustrated in Fig. 1 of JP 58-86075 A does not employ the one-way system, the koji making apparatus prevents contamination of miscellaneous bacteria from the outside of the apparatus by circulating air within a sealed circulation system, without introducing ambient air (page 2, lower left column, lines 11-14). In this koji making apparatus, a blower and an air conditioner are connected to a koji making room, and a cooler, a heater, a humidifier and an eliminator are arranged in the air conditioner in this order (page 2, lower left column, lines 18-20, Fig. 1).

In this air conditioner, the air that has passed through a koji layer and whose temperature is increased will be cooled by the cooler and then humidified by the humidifier, or will be cooled by the cooler and thereafter heated by the heater, and will be subsequently humidified by the humidifier (page 2, lower right column, lines 1-4). In this air conditioning control, when dehumidifying and drying are required, relative humidity can be reduced by reducing relative humidity through heating the air by the heater, or reducing absolute humidity through cooling the air by the cooler, and further reducing relative humidity through heating the air by the heater (page 2, lower right column, lines 7-12).

### SUMMARY

However, in a one-way system culture apparatus in which all the introduced ambient air passes through a culture material and is discharged, such as the culture apparatuses described in JP 9-154569 A and JP 4316021 B, it is difficult to adjust the temperature and the humidity, since the ambient air must be adjusted at once to set values for temperature and humidity, while adapting to variation in the ambient air. This problem becomes more disadvantageous when a size of the apparatus is increased, and there is also a problem that, in the control of adjusting the ambient air to the set values for temperature and humidity at once, the burden on a regulation unit is increased, and the running cost and the initial cost are increased.

Additionally, in the koji making apparatus described in JP 58-86075 A, there is a risk of energy loss, since the control of heating after cooling and the control of humidifying after dehumidifying are included.

The present invention solves the conventional problems as described above, and an object thereof is to provide an air flow type solid state culture apparatus and an air flow type solid state culture method that provide precise control as well as quick response, and are excellent in controllability.

In order to achieve the aforementioned object, an air flow type solid state culture apparatus of the present invention is an air flow type solid state culture apparatus that cultures microorganisms while adjusting temperature and humidity of ambient air taken into the apparatus, ventilating adjusted air that has been adjusted to a culture substrate, and thereafter exhausting all the air that has passed through the culture substrate to outside of the apparatus, in which the air flow type solid state culture apparatus includes an air intake, a blower, an air conditioning mechanism, a culture room, an air outlet, and a control device, the air conditioning mechanism is constituted by a cooling unit, a heating unit, a water spray unit, and a steam supply unit, for each of the units constituting the air conditioning mechanism, air conditioning control by the control device associates a combination of the units suitable for causing a state of temperature and humidity of pre-adjustment air to reach set values for temperature and humidity of the adjusted air with the state of the temperature and humidity of the pre-adjustment air for each of the set values, when the set values for the temperature and humidity of the adjusted air are specified, among the units constituting the air conditioning mechanism, the control device selects a combination of the units suitable for causing the temperature and humidity of the pre-adjustment air to reach the set values, based on the state of the pre-adjustment air, and the selected combination of the units is controlled by the control device to adjust the temperature and humidity of the pre-adjustment air.

An air flow type solid state culture method of the present invention is an air flow type solid state culture method using an air flow type solid state culture apparatus that cultures microorganisms while adjusting temperature and humidity of ambient air taken into the apparatus, ventilating adjusted air that has been adjusted to a culture substrate, and thereafter exhausting all the air that has passed through the culture substrate to outside of the apparatus, in which the air flow type solid state culture apparatus includes an air intake, a blower, an air conditioning mechanism, a culture room, an air outlet, and a control device, the air conditioning mechanism is constituted by a cooling unit, a heating unit, a water spray unit, and a steam supply unit, for each of the units constituting the air conditioning mechanism, air conditioning control by the control device associates a combination of the units suitable for causing a state of temperature and humidity of pre-adjustment air to reach set values for temperature and humidity of the adjusted air with the state of the temperature and humidity of the pre-adjustment air for each of the set values, when the set values for the temperature and humidity of the adjusted air are specified, among the units constituting the air conditioning mechanism, a combination of the units suitable for causing the temperature and humidity of the pre-adjustment air to reach the set values is selected by the control device based on the state of the pre-adjustment air, and the selected combination of the units is controlled by the control device to adjust the temperature and humidity of the pre-adjustment air.

According to the air flow type solid state culture apparatus and the air flow type solid state culture method of the present invention, when the set values for the temperature and humidity of the adjusted air are specified, among the units constituting the air conditioning mechanism, a combination of the units suitable for causing the pre-adjustment air to reach the set values is operated. Thus, when the state of the pre-adjustment air changes, or when the set values for the temperature and humidity of the adjusted air are changed, the combination is changed to a combination of the units suitable for the change. Therefore, it is possible to efficiently adjust the pre-adjustment air that has been taken in to reach the set values for the temperature and humidity of the adjusted air. Accordingly, the air conditioning control according to the present invention provides precise control as well as quick response, and is excellent in controllability. Additionally, since it is excellent in controllability, the energy loss is decreased, and the running cost and the initial cost can be reduced. Further, the steam supply unit having the effects of heating and humidification is provided for adjustment of the adjusted air to be supplied to the culture substrate. Thus, in a case where the heating unit is used as a unit for temperature adjustment, when the steam supply unit is used as a unit for humidity adjustment, the burden on the heating unit can be reduced. This can also reduce the running cost and the initial cost.

In the air flow type solid state culture apparatus and the air flow type solid state culture method of the present invention, it is preferable that the combination of the units is any one of a combination of the heating unit for temperature adjustment and the steam supply unit for humidity adjustment, a combination of the water spray unit for temperature adjustment and the steam supply unit for humidity adjustment, a combination of the cooling unit for temperature adjustment and the water spray unit for humidity adjustment, and a combination of the cooling unit for temperature adjustment and the heating unit for humidity adjustment.

According to this configuration, a total of two units, i.e., one unit for temperature adjustment and one unit for humidity adjustment, are selected. Therefore, control without excesses and deficiencies can be performed, it becomes possible to minimize the amount of usage of each of the units to efficiently reach the set values for the temperature and humidity of the adjusted air, and it is possible to further increase the effect of reducing the energy loss.

The effects of the present invention are as described above, and when the set values for the temperature and humidity of the adjusted air are specified, among the units constituting the air conditioning mechanism, a combination of the units suitable for causing the pre-adjustment air to reach the set values is operated. Thus, when the state of the pre-adjustment air changes, or when the set values for the temperature and humidity of the adjusted air are changed, the combination is changed to a combination of the units suitable for the change. Therefore, it is possible to efficiently adjust the pre-adjustment air that has been taken in to reach the set values for the temperature and humidity of the adjusted air. Accordingly, the air conditioning control according to the present invention provides precise control as well as quick response, and is excellent in controllability. Additionally, since it is excellent in controllability, the energy loss is decreased, and the running cost and the initial cost can be reduced. Further, the steam supply unit having the effects of heating and humidification is provided for adjustment of the adjusted air to be supplied to the culture substrate. Thus, in a case where the heating unit is used as a unit for temperature adjustment, when the steam supply unit is used as a unit for humidity adjustment, the burden on the heating unit can be reduced. This can also reduce the running cost and the initial cost.

### BRIEF DESCRIPTION OF THE DRAWINGS

Fig. 1 is a vertical cross-sectional view of an air flow type solid state culture apparatus according to an embodiment of the present invention;
Fig. 2 is a block diagram illustrating air conditioning control by a control device according to the embodiment of the present invention;
Fig. 3 is a flowchart illustrating a flow of the air conditioning control by the control device according to the embodiment of the present invention; and
Fig. 4 is a diagram illustrating an example of a corresponding relationship between each position and a combination of each units constituting an air conditioning mechanism in the embodiment of the present invention.

### DETAILED DESCRIPTION OF THE EMBODIMENT

Hereinafter, an embodiment of the present invention will be described with reference to the drawings. The present invention adjusts temperature and humidity of ambient air taken in an apparatus by an air conditioning mechanism 4 (refer to Fig. 1). In the following embodiment, air before entering the air conditioning mechanism 4 is called "pre-adjustment air", and air that has exited the air conditioning mechanism 4 is called "adjusted air." Additionally, "controllability" refers to precision of control and a response of control. Thus, being excellent in controllability means that the control is precise and the response is quick.

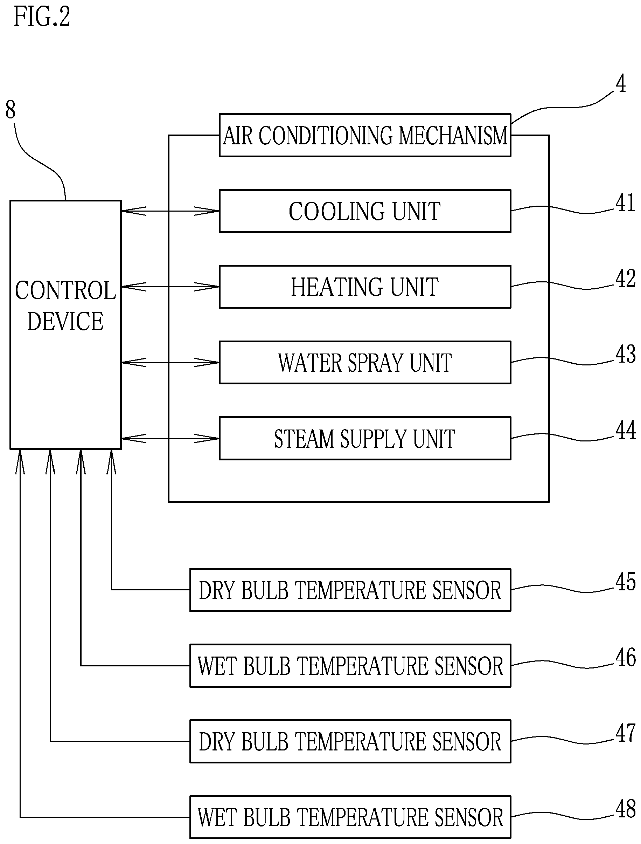

Fig. 1 illustrates a vertical cross-sectional view of an air flow type solid state culture apparatus 1 according to the embodiment of the present invention. The air flow type solid state culture apparatus 1 includes an air intake 2, a blower 3, the air conditioning mechanism 4, a culture room 5, an air outlet 6, a fan duct 7, and a control device 8 (refer to Fig. 2). The fan duct 7 corresponds to a duct after passing through the blower 3 to an inlet 54. The air conditioning mechanism 4 is in the fan duct 7, and is constituted by a cooling unit 41, a heating unit 42, a water spray unit 43, and a steam supply unit 44. Hereinafter, the units constituting the air conditioning mechanism is collectively called "the four units".

The culture room 5 is a space in a heat insulation housing 51. The culture room 5 is divided into an upper floor and a lower floor by a circular culture bed 52 that is horizontally rotatable. The culture bed 52 is formed with a perforated panel, and can perform ventilation for a culture substrate 53 deposited on the culture bed 52. The heat insulation housing 51 is connected to the inlet 54 for supplying the adjusted air whose temperature and humidity have been adjusted, and to an exhaust duct 56 for exhausting the air after ventilation. Additionally, a coarse dust filter and a sterilization filter are provided inside a duct that forms the air intake 2.

The air flow type solid state culture apparatus 1 cultures microorganisms while adjusting, by the four units 41 to 44, the temperature and humidity of the pre-adjustment air taken into the apparatus from the air intake 2, ventilating the adjusted air that has been adjusted to the culture substrate 53, and thereafter exhausting all the air that has passed through the culture substrate 53 to the outside of the apparatus. That is, an air conditioning control method of the air flow type solid state culture apparatus 1 is a one-way system in which all the adjusted air is exhausted to the outside of the apparatus after passing through the culture substrate, without circulating the adjusted air within the apparatus.

The temperature and humidity of the ambient air before being taken into the apparatus from the air intake 2 are measured by a dry bulb temperature sensor 47 and a wet bulb temperature sensor 48. The temperature and humidity of the adjusted air that have been adjusted by the four units 41 to 44 are measured by a dry bulb temperature sensor 45 and a wet bulb temperature sensor 46. A measured value of the dry bulb temperature sensor 47 is the temperature of the ambient air, and the humidity of the ambient air is calculated based on measured values of the dry bulb temperature sensor 47 and the wet bulb temperature sensor 48. A measured value of the dry bulb temperature sensor 45 is the temperature of the adjusted air, and the humidity of the adjusted air is calculated based on measured values of the dry bulb temperature sensor 45 and the wet bulb temperature sensor 46.

Measuring positions of the dry bulb temperature sensor 47 and the wet bulb temperature sensor 48 are outside the apparatus, and measuring positions of the dry bulb temperature sensor 45 and the wet bulb temperature sensor 46 are positions that allows measurement of the temperature and humidity of the adjusted air that has passed through the four units 41 to 44, or may be in the vicinity of the inlet 54 in the fan duct 7, or may be under the culture bed 52. These sensors are examples, and may be sensors capable of performing similar measurements.

Hereinafter, referring to Fig. 1, a specific description will be given of a flow of the air in the air flow type solid state culture apparatus 1 and a configuration of each part. When the blower 3 is operated, the pre-adjustment air is taken into the fan duct 7, and the air in the fan duct 7 is blown toward the culture room 5. The pre-adjustment air taken from the air intake 2 turns into the adjusted air whose temperature and humidity have been adjusted by the air conditioning mechanism 4 provided in the fan duct 7. As for the four units constituting the air conditioning mechanism 4, a combination of the units to be operated is selected by the control device 8 (refer to Fig. 2), and the units specified by the selected combination are controlled by the control device 8 to adjusts the temperature and humidity of the adjusted air. The details of this control will be described later.

The cooling unit 41 can adjust the blowing temperature by circulating chilled water in a heat exchanger 411, and cooling the air passing through the heat exchanger 411. Based on the measured values of the dry bulb temperature sensor 45 and the wet bulb temperature sensor 46, the control device 8 adjusts an amount of the chilled water to be supplied to the heat exchanger 411 to adjust the blowing temperature, by controlling opening of a control valve, which is an output value. The cooling unit 41 may have a different configuration as long as the air can be cooled.

The heating unit 42 supplies steam into the heat exchanger 421, and can heat the air passing through the heat exchanger 421 to adjust the blowing temperature. The control device 8 controls the opening of the control valve, which is an output value, based on the measured values of the dry bulb temperature sensor 45 and the wet bulb temperature sensor 46, thereby adjusting an amount of steam to be supplied to the heat exchanger 421 to adjust the blowing temperature or the blowing humidity.

The heating unit 42 may be, for example, an electric heater, as long as the air can be heated. In this case, the control device 8 controls a degree of demonstration (%) of performance of the electric heater, which is an output value, based on the measured values of the dry bulb temperature sensor 45 and the wet bulb temperature sensor 46, thereby adjusting the blowing temperature or the blowing humidity.

When using the steam as a heating source of the heating unit 42 as described above, a steam trap may be provided in the heat exchanger 421, or the heat exchanger 421 without a steam trap may be used. When the steam trap is not provided, an end of a steam pipe is opened to the atmosphere.

The water spray unit 43 includes a two-fluid nozzle 431, compressed air and water are supplied to the two-fluid nozzle 431, water is jetted out as a fine mist, and the air in the fan duct 7 can be humidified. Additionally, the effect of lowering the temperature of the air in the fan duct 7 can also be obtained by vaporization heat at the time of jetting. A one-fluid nozzle may be used instead of the two-fluid nozzle 431.

The water spray unit 43 is provided with an electromagnetic valve that can be opened and closed for each of the pipes for the compressed air and the water. The control device 8 simultaneously controls opening times of the two electromagnetic valves, which are output values, based on the measured values of the dry bulb temperature sensor 45 and the wet bulb temperature sensor 46, thereby adjusting the blowing temperature or the blowing humidity. For example, when a control cycle of spray water is 20 seconds, the opening time will be 10 seconds, which is 50% of 20 seconds, according to the measured values.

When there are a plurality of nozzles of the water spray unit 43, the control device 8 can also control the number of the nozzles for spraying, instead of the opening time, based on the measured values of the dry bulb temperature sensor 45 and the wet bulb temperature sensor 46. For example, when there are ten nozzles, the number of electromagnetic valves to be opened, which are provided in respective nozzles, will be three, which is 30% of the ten nozzles, according to the output value. Additionally, it is also effective to perform control by combining the control of the opening times of the electromagnetic valves and the control of the number of nozzles.

The steam supply unit 44 includes a steam supply nozzle 441, and can spray steam in the fan duct 7 to humidify the air in the fan duct 7, which also has the effect of heating the air at the same time. The control device 8 controls opening of the control valve, which is an output value, based on the measured values of the dry bulb temperature sensor 45 and the wet bulb temperature sensor 46, thereby adjusting an amount of the steam to be sprayed to adjust the blowing humidity.

The temperature and humidity of the adjusted air that has been subjected to the four units 41 to 44 are measured by the dry bulb temperature sensor 45 and the wet bulb temperature sensor 46, before entering the culture room 5. These measured values are sent to the control device 8 (refer to Fig. 2), and as will be described later in detail, the control device 8 controls each unit specified by a selected combination of the units, and performs adjustment of the temperature and humidity of the pre-adjustment air.

The adjusted air flows into the culture room 5 from the inlet 54 provided in the heat insulation housing 51, and thereafter passes through the culture substrate 53, and the air that has passed through the culture substrate 53 is exhausted to the outside of the apparatus from the air outlet 6 via an exhaust port 55 provided in the heat insulation housing 51 and the exhaust duct 56.

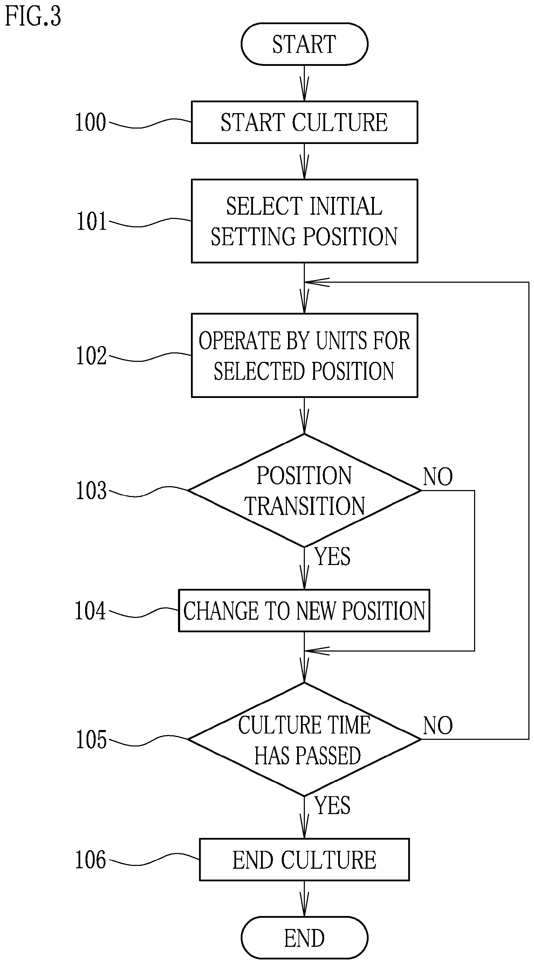

Hereinafter, air conditioning control of the air flow type solid state culture apparatus 1 will be described. Fig. 2 is a block diagram illustrating the air conditioning control by the control device 8. Fig. 3 is a flowchart illustrating a flow of the air conditioning control by the control device 8. The air conditioning control according to the present embodiment is control of adjusting the temperature and humidity of the pre-adjustment air taken into the apparatus to the set values for the temperature and humidity of the adjusted air. The set temperature is about 20 to 40°C, and the set humidity (relative humidity) is about RH90 to 98%. For example, by the air conditioning control according to the present embodiment, the pre-adjustment air with temperature and humidity of 15°C and RH60% is made into the adjusted air with temperature and humidity of 25°C and RH95%.

In the air conditioning control according to the present embodiment, for the four units 41 to 44 constituting the air conditioning mechanism 4, a combination of the units suitable for the state of the temperature and humidity of the pre-adjustment air to reach the set values for the temperature and humidity of the adjusted air is associated with the state of the temperature and humidity of the pre-adjustment air for each set value. The control device 8 selects a combination of the units to be operated from the four units 41 to 44 based on the set values for the temperature and humidity of the adjusted air, and the state of the temperature and humidity of the pre-adjustment air, and controls the selected combination of the units to adjust the temperature and humidity of the pre-adjustment air. The state of the temperature and humidity of the pre-adjustment air may be the measured values of the temperature and humidity of the pre-adjustment air, or may be that the temperature and humidity of the pre-adjustment air are within specific ranges. The latter can be determined based on an operation condition of each unit as will be describes later.

Although a manner or content of the association of the combinations of the units is not particularly limited, when the set values for the temperature and humidity of the adjusted air are specified, a combination of the units suitable for reaching the set values may be associated with the state of the temperature and humidity of the pre-adjustment air. In this case, when the control device 8 recognizes the set values for the temperature and humidity of the adjusted air, and the state of the temperature and humidity of the pre-adjustment air, the control device 8 will select a combination of the units corresponding to this condition.

The present inventors have derived association of the combination of the units based on positional division. A position refers to each region obtained by dividing a coordinate plane defined by temperature and humidity into a plurality of regions for each of the set values for the temperature and humidity of the adjusted air. Each position is associated with a combination of the specific units selected from the four units 41 to 44. In this case, assuming that the state of the temperature and humidity of the pre-adjustment air belongs to a certain position, by operating the combination of the units associated with this position, the temperature and humidity of the pre-adjustment air will be adjusted to be the set values for the temperature and humidity of the adjusted air.

As described above, since positional division is defined for each of the set values for the temperature and humidity of the adjusted air, when a set value is different, the positional divisions will also be different. Accordingly, when the set values for the temperature and humidity of the adjusted air are input to the control device 8, the control device 8 will select a combination of the units based on the state of the temperature and humidity of the pre-adjustment air, according to the positional division corresponding to the set values.

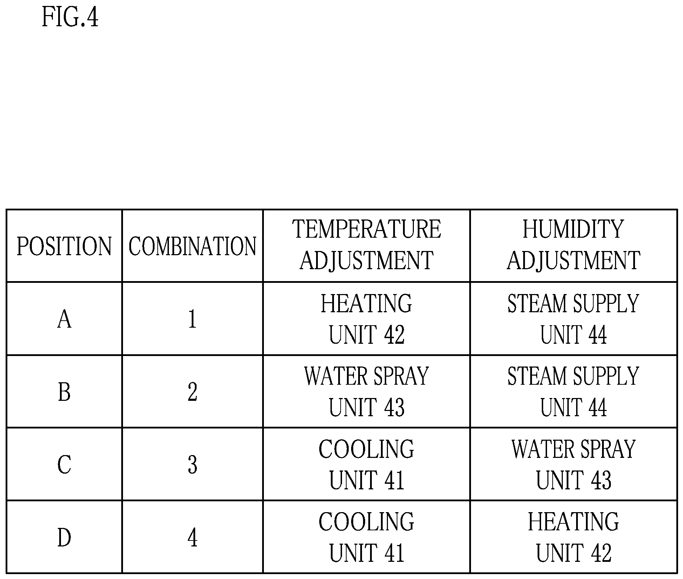

An example of the positional division will be described with reference to Fig. 4. Fig. 4 is a diagram illustrating an example of the corresponding relationship between each position and a combination of the units 41 to 44 constituting the air conditioning mechanism 4. As illustrated in Fig. 4, it is assumed that a region in the state of the pre-adjustment air that can be adjusted to the set values for the temperature and humidity of the adjusted air by the combination of the heating unit 42 for temperature adjustment and the steam supply unit 44 for humidity adjustment is a position A, a region in the state of the pre-adjustment air that can be adjusted to the set values for the temperature and humidity of the adjusted air by the combination of the water spray unit 43 for temperature adjustment and the steam supply unit 44 for humidity adjustment is a position B, a region in the state of the pre-adjustment air that can be adjusted to the set values for the temperature and humidity of the adjusted air by the combination of the cooling unit 41 for temperature adjustment and the water spray unit 43 for humidity adjustment is a position C, and a region in the state of the pre-adjustment air that can be adjusted to the set values for the temperature and humidity of the adjusted air by the combination of the cooling unit 41 for temperature adjustment and the heating unit 42 for humidity adjustment is a position D.

As illustrated in Fig. 4, a combination of the units is associated with each of the positions A to D. Thus, during the air conditioning control, the control device 8 selects a combination of the units selected from the four units 41 to 44, according to the positions A to D. In the example of Fig. 4, two units are selected from the four units 41 to 44 for each position. However, three or more units may be selected from the four units 41 to 44 as will be described later.

As described above, the positional division is defined for each of the set values of the temperature and humidity of the adjusted air. Therefore, in the example of the positions A to D, when set values are different, the regions of the positions A to D may be different, and a corresponding combination of the units may also be different.

Accordingly, when the set values of the temperature and humidity of the adjusted air are specified, the corresponding positional division is specified. Then, when the state of the temperature and humidity of the current pre-adjustment air is known, it may be determined to which position this state belongs. As a direct method of determining the current position, the temperature and humidity of the ambient air can be measured, and it can be determined to which position the measured value belongs. This will be described later in detail.

As another method of determining the current position, there is a method of performing the determination based on an output value, which is an operation condition of the units that are used at each position. The "output value" of a unit is a value of percentage of the performance of the unit to be demonstrated. Specifically, as described above, the output value of the cooling unit 41 is the opening of the control valve. The output value of the heating unit 42 is the opening of the control valve, or the degree of demonstration of the performance of the electric heater. The output value of the water spray unit 43 is the opening time of the electromagnetic valve, or the number of the nozzles for spraying. The output value of the steam supply unit 44 is the opening of the control valve.

When the method of determining the current position based on the output value is employed, an initial setting position may be forcibly selected at the beginning of culture. In this case, each unit specified in the combination for the initial setting position is controlled to adjust the temperature and humidity of the pre-adjustment air (steps 100 to 102 in Fig. 3). Based on the output values of the specified units, when it is determined that the initial setting position is not a position suitable for the state of the actual pre-adjustment air, it is determined that the position should be transitioned, and the position is changed to an appropriate position (steps 103 and 104 in Fig. 3).

The determination method for position transition is as follows. When the output value of one of the two units used for each position becomes smaller than a constant value, it can be presumed that the state of the pre-adjustment air or the set values for the temperature and humidity, which does not require the adjustment effect of the temperature and humidity by the unit, is achieved, and it is determined that the position is transitioned (step 103 in Fig. 3).

A description will be given of a case where the set values for the temperature and humidity of the adjusted air are constant, and the position is transitioned due to a change in the state of the pre-adjustment air caused by a change in the temperature and humidity of the ambient air. It is assumed that the state of the pre-adjustment air with respect to the set values for the temperature and humidity is the position A in Fig. 4, and the operation is performed by the combination 1. Additionally, it is assumed that, as the state of the pre-adjustment air changes, the output value of the heating unit 42 for temperature adjustment becomes smaller than a constant value. At this time, it can be presumed that the state of the pre-adjustment air is obtained that does not require the heating effect, which is the effect of the heating unit 42, and the control device 8 determines that the transition from the position A to the position B has been made (step 103 in Fig. 3).

Since the transition to the position B has been made, the control device 8 selects the combination 2 corresponding to the position B in Fig. 4 (step 104 in Fig. 3). This starts the operation of the water spray unit 43 for temperature adjustment and the steam supply unit 44 for humidity adjustment that are specified by the combination 2 (step 102 in Fig. 3).

It is assumed that, when the operation of the combination 2 is performed at the position B, as the state of the pre-adjustment air changes, the output value of the steam supply unit 44 for humidity adjustment becomes smaller than a constant value. At this time, it can be presumed that the state of the pre-adjustment air is obtained that does not require humidification by the steam supply unit 44, and the control device 8 determines that the transition from the position B to the position C has been made (step 103 in Fig. 3).

Since the transition to the position C has been made, the control device 8 selects the combination 3 corresponding to the position C in Fig. 4 (step 104 in Fig. 3). This starts the operation of the cooling unit 41 for temperature adjustment and the water spray unit 43 for humidity adjustment that are specified by the combination 3 (step 102 in Fig. 3).

Next, a description will be given of a case where the operation returns to the position B while the operation is performed by the combination 3 at the position C. It is assumed that, as the state of the pre-adjustment air changes, the output value of the cooling unit 41 for temperature adjustment becomes smaller than a constant value. At this time, it can be presumed that the state of the pre-adjustment air is obtained that does not require the cooling effect, which is the effect of the cooling unit 41, and the control device 8 determines that the transition from the position C to the position B has been made (step 103 in Fig. 3).

Since the transition to the position B has been made, the control device 8 selects the combination 2 corresponding to the position B in FIG. 4 (step 104 in Fig. 3). This starts the operation of the water spray unit 43 for temperature adjustment and the steam supply unit 44 for humidity adjustment that are specified by the combination 2 (step 102 in Fig. 3).

Next, a description will be given of a case where the state of the pre-adjustment air is constant, and when the position is transitioned due to a change in the set values for the temperature and humidity of the adjusted air. The culture substrate 53 generates heat over time, and in order to control the temperature of the culture substrate, the set values for the temperature and humidity of the adjusted air are changed. It is assumed that the state of the pre-adjustment air with respect to the set values for the temperature and humidity of the current adjusted air is the position A in FIG. 4, and the operation is performed by the combination 1. Additionally, it is assumed that, as the set values for the temperature and humidity of the adjusted air change, the output value of the heating unit 42 for temperature adjustment becomes smaller than a constant value. At this time, it can be presumed that the state of the adjusted air is obtained that does not require the heating effect, which is the effect of the heating unit 42, and the control device 8 determines that the transition from the position A to the position B has been made (step 103 in Fig. 3).

Since the transition to the position B has been made, the control device 8 selects the combination 2 corresponding to the position B in Fig. 4 (step 104 in Fig. 3). This starts the operation of the water spray unit 43 for temperature adjustment and the steam supply unit 44 for humidity adjustment that are specified by the combination 2 (step 102 in Fig. 3). As described above, when the set values for the temperature and humidity of the adjusted air change, as well as when the state of the pre-adjustment air changes, the transition of the position can be determined based on the output values of the units that are used at each position.

Although the description is given above of the method of determining the current position based on the output values of the units that are used, the temperature and humidity of the ambient air may be measured, and it may be determined to which position the current ambient air state belongs. As described above, when the set values for the temperature and humidity of the adjusted air are specified, the corresponding positional division is specified. Thus, by measuring the temperature and humidity of the current ambient air, it can be directly determined to which position the measured values belong. The temperature and humidity of the ambient air taken into the apparatus are measured by the dry bulb temperature sensor 47 and the wet bulb temperature sensor 48 that are illustrated in Fig. 1, and are sent to the control device 8 (Fig. 2).

In any case of the method of determining the current position based on the output values of the units, or the method of determining the current position by measuring the temperature and humidity of the ambient air, whenever the position is transitioned, steps 102 to 105 in Fig. 3 are repeated, and a combination is selected. Accordingly, even when there is a change in the set values for the temperature and humidity or a variation in the state of the pre-adjustment air during culture, each unit specified by the combination is controlled by the control device 8, and the temperature and humidity of the adjusted air can be adjusted.

In the aforementioned embodiment, the operation is performed by selecting a combination of the units that are selected from the four units 41 to 44 and that are suitable for the position, and whenever the position is transitioned, a combination suitable for the transitioned position will be selected to adjust the temperature and humidity of the pre-adjustment air. In this case, when the state of the pre-adjustment air changes during the operation by the combination of the units suitable for the position, or when the set values for the temperature and humidity of the adjusted air are changed, a combination of the units suitable for a new position will be selected, and the state of the pre-adjustment air will be efficiently adjusted so as to reach the set values for the temperature and humidity of the adjusted air.

Additionally, in the aforementioned embodiment, the steam supply unit 44 is provided, and steam atomizing has the effects of heating and humidification. Therefore, in a case where the heating unit 42 is used as a unit for temperature adjustment, when the steam supply unit 44 is used as a unit for humidity adjustment (refer to the combination 1 in Fig. 4), the burden on the heating unit 42 can be reduced. This can also reduce the running cost and the initial cost.

Although the embodiment of the air conditioning control is described above, in the air conditioning control according to the present invention, when the set values for the temperature and humidity of the adjusted air are specified, among the units constituting the air conditioning mechanism 4, the control device 8 selects a combination of the units suitable for reaching the set values, based on the state of the pre-adjustment air. In this air conditioning control, when the state of the pre-adjustment air changes, or when the set values for the temperature and humidity of the adjusted air are changed, the combination is changed to a combination of the units suitable for the change. Thus, the pre-adjustment air that has been taken in can be efficiently adjusted to be the set values for the temperature and humidity of the adjusted air. Therefore, the air conditioning control according to the present invention provides precise control as well as quick response, and is excellent in controllability. Since it is excellent in controllability, the energy loss is decreased, and the running cost and the initial cost can also be reduced.

Further, in the combinations illustrated in Fig. 4, among the four units 41 to 44, irrespectively of the position, a total of two units, i.e., one unit for temperature adjustment and one unit for humidity adjustment, are selected as the units to be used. Therefore, control without excesses and deficiencies can be performed, it becomes possible to minimize the amount of usage of each of the units to reach the set values for the temperature and humidity of the adjusted air, and it is possible to further increase the effect of reducing the energy loss.

The number of units to be selected from the four units 41 to 44 is not limited to two, and may be three or more. Even when the number is three or more, combinations of the units can be formed, and the same effects as in the case where the number is two can be obtained. As an example of using three units, a description will be given of a case where the operation is performed at the position B. Although the position B uses two units, i.e., the water spray unit 43 and the steam supply unit 44 in the combination in Fig. 4, the control can be performed by three units, i.e., by adding the cooling unit 41 to the above-described two units. At this time, the output value of the cooling unit 41 is set to a fixed value, and the water spray unit 43 for temperature adjustment and the steam supply unit 44 for humidity adjustment are controlled to adjust the temperature and humidity of the adjusted air.

As described above, the air conditioning control according to the present invention is excellent in the controllability of temperature and humidity. However, the controllability is further increased by appropriately changing the selection of a combination of the units. The combinations illustrated in Fig. 4 is an example selected from this viewpoint. Specifically, although some of the four units 41 to 44 may be used for temperature adjustment as well as humidity adjustment, when they are used, the effect on temperature may be different from the effect on humidity. For example, the steam supply unit 44 has a large effect on humidity, but has a small effect on temperature. Therefore, in the combinations illustrated in Fig. 4, the steam supply unit 44 is selected for humidity adjustment (the combinations 1 and 2), and is not selected for temperature adjustment. That is, in the combinations illustrated in Fig. 4, after considering the effect on temperature and the effect on humidity of each unit, a unit suitable for temperature adjustment is selected as the unit for temperature adjustment, and a unit suitable for humidity adjustment is selected as the unit for humidity adjustment. Thus, the controllability is further increased.

Although one embodiment of the present invention is described above, the above-described embodiment may be appropriately varied. For example, in the air flow type solid state culture apparatus 1 illustrated in Fig. 1, an arrangement order of the water spray unit 43 and the steam supply unit 44 may be reversed, and the blower 3 may be arranged downstream of the heating unit 42.

### Reference Signs List

- 1: air flow type solid state culture apparatus
- 2: air intake
- 3: blower
- 4: air conditioning mechanism
- 5: culture room
- 6: air outlet
- 7: fan duct
- 8: control device
- 41: cooling unit
- 42: heating unit
- 43: water spray unit
- 44: steam supply unit
- 45: dry bulb temperature sensor
- 46: wet bulb temperature sensor
- 47: dry bulb temperature sensor
- 48: wet bulb temperature sensor
- 51: heat insulation housing
- 52: culture bed
- 53: culture substrate
- 54: inlet port
- 55: exhaust port
- 56: exhaust duct
- 411: heat exchanger
- 421: heat exchanger
- 431: two-fluid nozzle
- 441: steam supply nozzle

## Claims

1. An air flow type solid state culture apparatus (1) that cultures microorganisms while adjusting temperature and humidity of ambient air taken into the apparatus (1), ventilating adjusted air that has been adjusted to a culture substrate (53), and thereafter exhausting all the air that has passed through the culture substrate (53) to outside of the apparatus (1),
wherein the air flow type solid state culture apparatus (1) comprises an air intake (2), a blower (3), an air conditioning mechanism (4), a culture room (5), an air outlet (6), and a control device (8),
the air conditioning mechanism (4) is constituted by a cooling unit (41), a heating unit (42), a water spray unit (43), and a steam supply unit (44),
for each of the units constituting the air conditioning mechanism (4), air conditioning control by the control device (8) associates a combination of the units suitable for causing a state of temperature and humidity of pre-adjustment air to reach set values for temperature and humidity of the adjusted air with the state of the temperature and humidity of the pre-adjustment air for each of the set values,
when the set values for the temperature and humidity of the adjusted air are specified, among the units constituting the air conditioning mechanism (4), the control device (8) selects a combination of the units suitable for causing the temperature and humidity of the pre-adjustment air to reach the set values, based on the state of the pre-adjustment air, and
the selected combination of the units is controlled by the control device (8) to adjust the temperature and humidity of the pre-adjustment air.

2. The air flow type solid state culture apparatus (1) according to claim 1, wherein the combination of the units is any of a combination of the heating unit (42) for temperature adjustment and the steam supply unit (44) for humidity adjustment, a combination of the water spray unit (43) for temperature adjustment and the steam supply unit (44) for humidity adjustment, a combination of the cooling unit (41) for temperature adjustment and the water spray unit (43) for humidity adjustment, and a combination of the cooling unit (41) for temperature adjustment and the heating unit (42) for humidity adjustment.

3. An air flow type solid state culture method using an air flow type solid state culture apparatus (1) that cultures microorganisms while adjusting temperature and humidity of ambient air taken into the apparatus (1), ventilating adjusted air that has been adjusted to a culture substrate (53), and thereafter exhausting all the air that has passed through the culture substrate (53) to outside of the apparatus (1),
wherein the air flow type solid state culture apparatus (1) comprises an air intake (2), a blower (3), an air conditioning mechanism (4), a culture room (5), an air outlet (6), and a control device (8),
the air conditioning mechanism (4) is constituted by a cooling unit (41), a heating unit (42), a water spray unit (43), and a steam supply unit (44),
for each of the units constituting the air conditioning mechanism (4), air conditioning control by the control device (8) associates a combination of the units suitable for causing a state of temperature and humidity of pre-adjustment air to reach set values for temperature and humidity of the adjusted air with the state of the temperature and humidity of the pre-adjustment air for each of the set values,
when the set values for the temperature and humidity of the adjusted air are specified, among the units constituting the air conditioning mechanism (4), a combination of the units suitable for causing the temperature and humidity of the pre-adjustment air to reach the set values is selected by the control device based on the state of the pre-adjustment air, and
the selected combination of the units is controlled by the control device (8) to adjust the temperature and humidity of the pre-adjustment air.

4. The air flow type solid state culture method according to claim 3, wherein the combination of the units is any of a combination of the heating unit (42) for temperature adjustment and the steam supply unit (44) for humidity adjustment, a combination of the water spray unit (43) for temperature adjustment and the steam supply unit (44) for humidity adjustment, a combination of the cooling unit (41) for temperature adjustment and the water spray unit (43) for humidity adjustment, and a combination of the cooling unit (41) for temperature adjustment and the heating unit (42) for humidity adjustment.
